Europäisches Patentamt

European Patent Office

Office européen des brevets

(19)

(11) Veröffentlichungsnummer: **0 192 971**
**B1**

(12) **EUROPÄISCHE PATENTSCHRIFT**

(45) Veröffentlichungstag der Patentschrift: 04.04.90

(21) Anmeldenummer: 86101010.6

(22) Anmeldetag: 25.01.86

(51) Int. Cl.⁵: **A 61 F 7/00,** F 25 B 19/00,
F 25 D 3/10

(54) Vorrichtung zur Erzeugung eines trockenen kalten Gasstromes.

(30) Priorität: 27.02.85 DE 3506932

(43) Veröffentlichungstag der Anmeldung:
03.09.86 Patentblatt 86/36

(45) Bekanntmachung des Hinweises auf die
Patenterteilung:
04.04.90 Patentblatt 90/14

(84) Benannte Vertragsstaaten:
AT BE CH FR GB IT LI NL SE

(56) Entgegenhaltungen:
DE-A-2 839 214
DE-A-3 242 881

(73) Patentinhaber: MESSER GRIESHEIM GMBH
Hanauer Landstrasse 330
D-6000 Frankfurt/Main (DE)

(72) Erfinder: Thoma, Klemens
Am Kleckers 22
D-4150 Krefeld-Hüls 29 (DE)
Erfinder: Volker, Wolfgang
Pastorsbusch 35
D-4154 Tönisvorst 1 (DE)

Courier Press, Leamington Spa, England.

**Beschreibung**

Die Erfindung betrifft eine Vorrichtung zur Erzeugung eines trockenen kalten Gasstromes durch Verdampfen eines tiefsiedenden verflüssigten Gases für die Kältetherapie rheumatischer Erkrankungen.

Für die Kältetherapie rheumatischer Erkrankgungen hat sich in den vergangenen Jahren flüssiger Stickstoff als Kältemittel mehr und mehr durchgesetzt. Vielfach wird hierbei verdampfter flüssiger Stickstoff mit Luft oder Sauerstoff vermischt, um ein Behandlungsgas der gewünschten Temperatur zu erhalten. Entsprechende Vorrichtungen zeigen die Deutschen Offenlegungsschriften 32 42 881 und 28 39 214. Generell muß bei diesen Einrichtungen sichergestellt werden, daß keine Feuchtigkeit aus der umgebenden Atmosphäre ausfriert und mit dem Behandlungsgas auf die zu behandelnden Körperpartien gelangt. Außerdem darf es nicht zu einer gefährlichen Sauerstoffverarmung durch Verdampfen von zu viel flüssigem Stickstoff kommen. Zur Beseitigung der Feuchtigkeit und dem Aufrechterhalten eines ausreichenden Sauerstoffgehaltes sind verhältnismäßig aufwendige Einrichtungen erforderlich. Die Behandlungsgeräte werden hierdurch so teuer und voluminös, daß sich ihr Einsatz nur in speziellen Therapienzentren lohnt. Hier erfüllen sie allerdings ihren Zweck zufriedenstellend.

Es besteht aber das Bedürfnis, die Kältetherapie rheumatischer Erkrankungen nicht auf spezielle Therapiezentren zu beschränken, sondern auch in durchschnittlichen Arztpraxen und Krankenhäusern zu ermöglichen.

Der Erfindung liegt daher die Aufgabe zugrunde, eine Vorrichtung für die Kältetherapie rheumatischer Erkrankungen mit flüssigem Stickstoff als Kältemedium zu schaffen, welche so leicht und einfach aufgebaut ist, daß sie auch als mobiles Gerät ausgebildet werden kann.

Ausgehend von dem im Oberbegriff des Anspruches 1 berücksichtigten Stand der Technik (DE-A-2 839 214) ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Das beschriebene Ausführungsbeispiel der Erfindung besitzt zwei wesentliche Merkmale. Das eine Merkmal besteht darin, als Behandlungsgas auschließlich verdampften flüssigen Stickstoff zu verwenden. Dieser Stickstoff ist vollkommen trokken, so daß keine Probleme hinsichtlich ausfrierender Feuchtigkeit entstehen können.

Um die gewünschte Behandlungstemperatur zu erreichen, ist jedoch eine zweifache geregelte Heizung erforderlich, nämlich einmal zum Verdampfen des Stickstoffes und danach zum Aufheizen des verdampften Stickstoffes auf die gewünschte Behandlungstemperatur. Das zweite wesentliche Merkmal besteht darin, das Speichervolumen des Gefäßes zur Aufnahme des tiefsiedenden verflüssigten Gases also, normalerweise von flüssigem Stickstoff, so gering zu machen, daß selbst bei vollständiger Verdampfung des verflüssigten Gases keine unzulässige Verarmung an Sauerstoff eintreten kann. Bei Stickstoff ist hierbei ein Speichervolumen von max. 6 Litern angebracht. Selbst bei vollständiger Verdampfung von 6 Litern flüssigen Stickstoffs in einem Krankenzimmer tritt nämlich keine gefährliche Verringerung des Sauerstoffgehaltes ein. Andererseits reichen 6 Liter, um 3 bis 6 Patienten behandeln zu können.

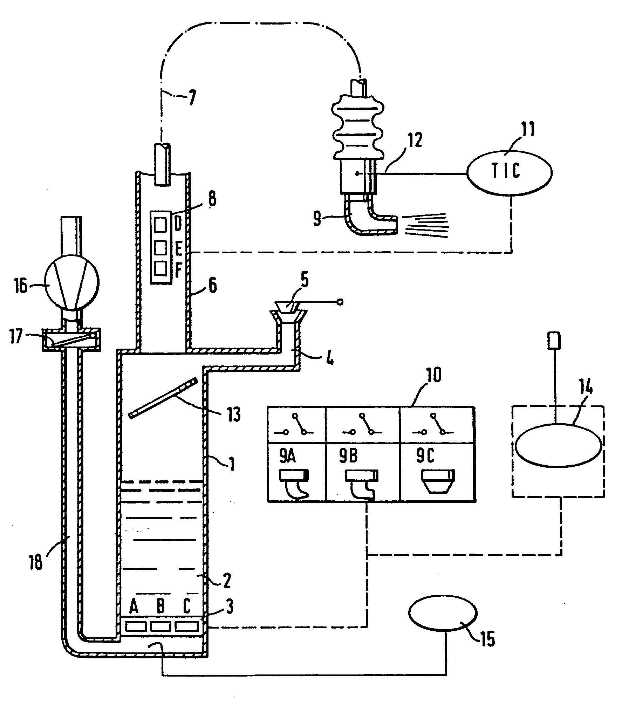

Die Zeichnung veranschaulicht ein Ausführungsbeispiel der erfindungsgemäßen Vorrichtung in schematischer Form.

Die Vorrichtung besteht aus einem Gefäß 1 zur Aufnahme des flüssigen Stickstoffes 2. Am Boden des Gefäßes 1 befindet sich eine elektrische Heizung 3, deren Leistung durch Zu- bzw. Abschalten der Heizwicklungen A, B und C reguliert werden kann. Das Gefäß 1 wird mittels einer Tauchpumpe durch den Stutzen 4 mit flüssigem Stickstoff gefüllt. Der Stutzen 4 wird durch ein Kegelventil 5 verschlossen. Die Dichtung des Kegelventils 5 wird von einem Dauermagneten gegen den Überdruck im dem System von ca. 50 mbar gehalten.

An den Gasraum des Gefäßes 1 ist eine Heizstrecke 6 angeschlossen, von der eine Schlauchleitung 7 weitergeführt wird. In der Heizstrecke 6 befindet sich ein elektrisches Heizregister 8, dessen Leistung durch Zu- bzw. Abschalten der Heizwicklungen D, E und F regelbar ist. Die Schlauchleitung 7 endet in der auswechselbaren Behandlungsdüse 9. Die auswechselbaren Behandlungsdüsen 9 haben verschiedene Querschnitte. Um zu erreichen, daß die Geschwindigkeit des austretenden Gasstromes bei allen Düsen annähernd gleich ist, sind für die einzelnen Behandlungsdüsen 9 unterschiedliche Heizleistungen der elektrischen Heizung 3 erforderlich. Die jeweils erforderliche Heizleistung wird beim Einsetzen der Behandlungsdüsen 9 durch eine elektrische Verbindung automatisch eingestellt. Dies ist symbolisch durch den Block 10 mit den drei unterschiedlichen Behandlungsdüsen 9A, 9B und 9C dargestellt. In entsprechender Weise werden auch die Heizwicklungen des Heizregisters 8 zugeschaltet. Obwohl die Heizleistung des Heizregisters 8 viel geringer ist als die der elektrischen Heizung 3, ist auch hier eine Anpassung der Heizleistung an den Düsenquerschnitt erforderlich, weil sonst die Temperaturregelung überfordert würde. Zur Temperaturregelung dient der Temperaturfühler 12 im Bereich der Behandlungsdüse 9 und der eigentliche Temperaturregler 11, der auf das Heizregister 8 wirkt. Die Heizwicklungen D, E und F des Heizregisters 8 sind Drähte, die sich direkt im Stickstoffstrom befinden. Die träge Masse ist hierbei außerordentlich gering, die Ansprechzeit entsprechend kurz. Die Temperaturregelung ist daher außerordentlich schnell und exakt. Im Gasraum des Gefäßes 1 befindet sich noch eine Umlenkscheibe 13, welche das Mitreißen von Flüssigkeitstropfen in die Heizstrecke 6 verhindert.

Obwohl eine Sauerstoffüberwachung des Behandlungsraumes an sich nicht erforderlich ist, kann diese doch in einfacher Weise erfolgen, falls hierauf nicht verzichtet werden soll. Eine entsprechende Sauerstoff-Überwachungseinrichtung 14 wirkt dann auf die Steuerung in der Art, daß bei einem Grenzwert von weniger als 17 Vol.-% Sauerstoff die Heizung 3 und das Heizregister 8 abgeschaltet werden.

Nach dem Ende der Behandlung wird die gesamte Vorrichtung mit Raumluft durchblasen. Zuvor muß jedoch eventuell noch vorhandener flüssiger Stickstoff entfernt werden, weil sich an diesem Sauerstoff in gefährlicher Weise aufkonzentrieren könnte. Hierzu dient die Niveauüberwachung 15, welche erst beim Erreichen eines Grenzniveaus für den flüssigen Stickstoff 2 die Gebläseluft freigibt. Die Gebläseluft wird durch das Gebläse 16 angesaugt und über eine Klappe 17 und den Stutzen 18 durch das Gefäß 1, die Heizstrecke 6, die Schlauchleitung 7 und die Behandlungsdüse 9 geleitet. Die Klappe 17 verhindert, daß während der Therapie Kaltgas entweichen kann. Das Gerät wird dann so lange mit Raumluft durchströmt, bis es Umgebungstemperatur angenommen hat. Die Wiedererwärmung der Vorrichtung kann auch mit verdampftem flüssigen Stickstoff erfolgen, der im Kreislauf geführt wird.

Die erfindungsgemäße Vorrichtung wird vorzugsweise als mobile Einrichtung konzipiert. Sie wird zweckmäßigerweise so ausgelegt, daß ein Netzanschluß von 220V und 2 kw genügt. Die elektrische Heizung 3 kann dabei beispielsweise auf 0,9 kw, 1,35 kw und 1,8 kw abgestuft werden. Entsprechend weist das Heizregister 8 Leistungen von 100 W, 150 W und 200 W auf. Es ist zwar vorteilhaft, wenn die Zuordnung der Heizleistung zu den einzelnen Behandlungsdüsen automatisch erfolgt, die Vorrichtung kann aber auch ohne weiteres so gestaltet werden, daß die Einstellung der Heizleistung von Hand erfolgt. Für Sonderfälle kann auch die kontinuierliche Veränderung der Heizleistung vorgesehen werden. Ein Überfüllen des Gefäßes 1 kann durch einen Betriebszeitbegrenzer verhindert werden, der an die Tauchpumpe angeschlossen wird. Selbstverständlich können anstelle einer Tauchpumpe auch andere Abfüllsysteme eingesetzt werden, z.B. Behälter mit einem eigenen Druckaufbausystem, in welche über ein Magnetventil abgefüllt wird.

Die erfindungsgemäße Vorrichtung hat zwar verhältnismäßig hohe spezifische Betriebskosten, weil die Kälte des flüssigen Stickstoffs nur unvollkommen ausgenutzt wird, indem der gesamte Stickstoff auf die gewünschte Behandlungstemperatur elektrisch aufgeheizt wird. Da die Vorrichtung aber nur für gelegentliche Einzelbehandlungen und nicht für einen Dauerbetrieb konzipiert ist, fallen die etwas höheren Betriebskosten gegenüber den Vorteilen nicht ins Gewicht. Diese Vorteile sind ein unkomplizierter technischer Aufbau, Mobilität, Kleinheit und jederzeit vorhandene Einsatzbereitschaft.

**Patentansprüche**

1. Vorrichtung zur Erzeugung eines trockenen kalten Gasstromes durch Verdampfen eines tiefsiedenden verflüssigten Gases für die Kältetherapie rheumatischer Erkrankungen, mit einem Gefäß (1) zur Aufnahme des tiefsiedenden verflüssigten Gases und einer an das Gefäß angeschlossenen Schlauchleitung (7) mit auswechselbarer Behandlungsdüse (9) für die Abgabe des verdampften Gases, dadurch gekennzeichnet, daß das Gefäß nur ein geringes Speichervolumen besitzt, in der Nähe seines Bodens eine regelbare elektrische Heizung (3) zum Verdampfen des verflüssigten Gases aufweist und die Schlauchleitung mittels einer regelbaren elektrischen Heizstrecke (6) an das Gefäß angeschlossen ist.

2. Vorrichtung nach Anspruch 1, dadurch gekennzeichnet, daß die Leistung der elektrischen Heizungen (3, 8) im Gefäß durch Einsetzen der auswechselbaren Behandlungsdüse über eine elektrische Verbindung eingestellt wird.

3. Vorrichtung nach Anspruch 1 oder 2, dadurch gekennzeichnet, daß die Leistung der elektrischen Heizstrecke (6) durch einen Temperaturfühler (12) im Bereich der Behandlungsdüse geregelt wird.

4. Vorrichtung nach einem der Ansprüche 1 bis 3, dadurch gekennzeichnet, daß das maximale Speichervolumen des Gefäßes 6 Liter nicht wesentlich überschreitet.

**Revendications**

1. Dispositif pour obtenir un écoulement de gaz froid et sec par vaporisation d'un gaz liquéfié à basse température d'ébullition pour la cryothérapie d'affections rhumatismales, dispositif comportant un récipient (1) destiné à recevoir le gaz liquéfié à basse température d'ébullition et une canalisation souple (7) raccordée à ce récipient, et munie d'une buse de traitement (9) interchangeable pour délivrer le gaz obtenu par vaporisation, dispositif caractérisé en ce que le récipient n'a qu'un volume de stockage réduit, il comporte au voisinage de son fond, un chauffage électrique réglable (3) pour vaporiser le gaz liquéfié, et la canalisation souple est raccordée au récipient au moyen d'une section électrique de chauffe réglable (6).

2. Dispositif selon la revendication 1, caractérisé en ce que la puissance des chauffages électriques (3, 8) dans le récipient, est réglée par l'intermédiaire d'une liaison électrique du fait de la mise en place de la buse de traitement interchangeable.

3. Dispositif selon la revendication 1 ou 2, caractérisé en ce que la puissance de la section électrique de chauffe (6) est réglée par l'intermédiaire d'un détecteur de température (12) au voisinage de la buse de traitement.

4. Dispositif selon une des revendications 1 à 3, caractérisé en ce que le volume de stockage maximal du récipient ne dépasse pas en pratique 6 litres.

## Claims

1. Apparatus for generating a cold dry stream of gas by vaporizing a low-boiling liquified gas for the crymotherapy of rheumatic illnesses, having a vessel (1) for accommodating the low-boiling liquified gas and a hose pipe (7) which is connected to the vessel and has an exchangeable treatment nozzle (9) for delivering the vaporized gas, characterized in that the vessel has only a small storage volume, has in the vicinity of its floor a regulatable electric heater (3) for vaporizing the liquified gas, and the hose pipe is connected to the vessel via a regulatable electric heating section (6).

2. Apparatus according to Claim 1, characterized in that the output of the electric heaters (3, 8) in the vessel is set by inserting the exchangeable treatment nozzle via an electrical connection.

3. Apparatus according to Claim 1 or 2, characterized in that the output of the electric heating section (6) is regulated by means of a temperature probe (12) in the region of the treatment nozzle.

4. Apparatus according to one of Claims 1 to 3, characterized in that the maximum storage volume of the vessel does not greatly exceed 6 litres.